# EUROPÄISCHE PATENTSCHRIFT

(11) **EP 1 158 924 B1**
(45) Veröffentlichungstag und Bekanntmachung des Hinweises auf die Patenterteilung: **31.03.2004**
(21) Anmeldenummer: 00901590.0
(22) Anmeldetag: 24.01.2000
(51) Int. Cl.: A61B 19/00

(54) **AKTIVES INSTRUMENT ZUR POSITIONSBESTIMMUNG BEI NAVIGATIONSSYSTEMEN ZUR UNTERSTÜTZUNG CHIRURGISCHER EINGRIFFE**
ACTIVE INSTRUMENT FOR DETERMINING A POSITION IN NAVIGATION SYSTEMS FOR ASSISTING SURGICAL INTERVENTIONS
INSTRUMENT ACTIF POUR DETERMINER UNE POSITION DANS DES SYSTEMES DE GUIDAGE SERVANT A ASSISTER DES INTERVENTIONS CHIRURGICALES

(30) Priorität: 05.03.1999 DE 29904018 U
(43) Veröffentlichungstag der Anmeldung: 05.12.2001
(73) Patentinhaber: Northern Digital Inc., Waterloo, Ontario N2V 1C5 (CA)
(72) Erfinder: SCHMID, Manfred, D-88634 Herdwangen (DE); KONRAD, Peter, D-78333 Stockach (DE)
(74) Vertreter: Söltenfuss, Dirk Christian, Dipl.-Phys.
(86) Internationale Anmeldenummer: PCT/EP2000/000508
(87) Internationale Veröffentlichungsnummer: WO 2000/053115

(56) Entgegenhaltungen:
- WO-A-90/12541
- WO-A-95/25475
- DE-C- 4 225 112

## Beschreibung

Die Neuerung betrifft ein aktives Instrument zur Positionsbestimmung bei Navigationssystemen zur Unterstützung chirurgischer Eingriffe.

Es ist bekannt, während einer Operation die Lage eines chirurgischen Instruments oder eines Zeigeinstrumentes (nachfolgend Instrument genannt) im Körper eines Patienten für den Operateur auf einem Bildschirm sichtbar zu machen. Zu diesem Zweck werden vor der Operation Schichtbilder, z. B. ein Computertomogramm (CT) oder ein Kernspinntomogramm (MRI), des zu operierenden Körperteils des Patienten aufgenommen. Während der Operation wird mittels eines Meßsystems, z.B. mittels zweier Kameras, durch Auswertung der Lage von an dem Instrument angebrachten Lichtquellen die Position des Instruments im Raum bestimmt. Diese Lage wird zusammen mit den Schichtbildern auf einem Bildschirm dargestellt. Die bei der Operation bestehende Lage des Patienten oder des zu operierenden Körperteils wird durch Marken ermittelt, die sowohl während der Aufnahme der Schichtbilder als auch während der Operation an dem Patienten angebracht sind oder reproduzierbar angebracht werden können. Die Lage dieser Marken wird während der Operation bestimmt. Das kann dadurch geschehen, daß das Instrument zu diesen Marken hingeführt und seine Position bestimmt wird. Dadurch ist die Patientenposition im Raum bekannt und eine eindeutige Beziehung zwischen Patientenposition und Schichtbildern hergestellt.

Die Stromversorgung der Instrumente erfolgt bei bekannten "aktiven Instrumenten" dieser Art über ein Kabel. Ein solches Kabel ist störend und bietet Probleme bei der Sterilisierung des Bauteils.

Es ist auch bekannt z.B. aus WO-A-95/25475, für die Stromversorgung einen eingebauten Akkumulator vorzusehen. Zum Laden und zum Sterilisieren des Bauteils muß dieser Akkumulator ausgebaut werden.

Der Neuerung liegt die Aufgabe zu Grunde, bei einem aktiven Instrument zur Positionsbestimmung bei Navigationssystemen zur Unterstützung chirurgischer Eingriffe die Stromversorgung zu verbessern.

Neuerungsgemäß wird diese Aufgabe dadurch gelöst, daß das Instrument wenigstens eine Solarzelle zu seiner Stromversorgung trägt.

Bei Operationen ist üblicherweise das Operationsfeld sehr hell ausgeleuchtet. Diese Beleuchtung kann über die Solarzelle oder Solarzellen zur Erzeugung der in dem Instrument erforderlichen, relativ geringen elektrischen Leistungen, z.B. für die Speisung von Leuchtdioden, ausgenutzt werden.

Es ist auch möglich, daß das Bauteil einen von der Solarzelle aufladbaren Puffer (z. B. Kondensator) zur kurzfristigen Pufferung der Stromversorgung aufweist.

Das Instrument kann Lichtquellen zur Positionsbestimmung des Instrumentes aufweisen, die von der Stromversorgung gespeist werden. Das Instrument kann aber auch drahtlose Kommunikationsmittel aufweisen, die ebenfalls von der Stromversorgung gespeist werden. Diese Kommunikationsmittel können zur Identifizierung des Bauteils dienen oder zur Steuerung der Lichtquelle, z.B. zum Ein- und Ausschalten.

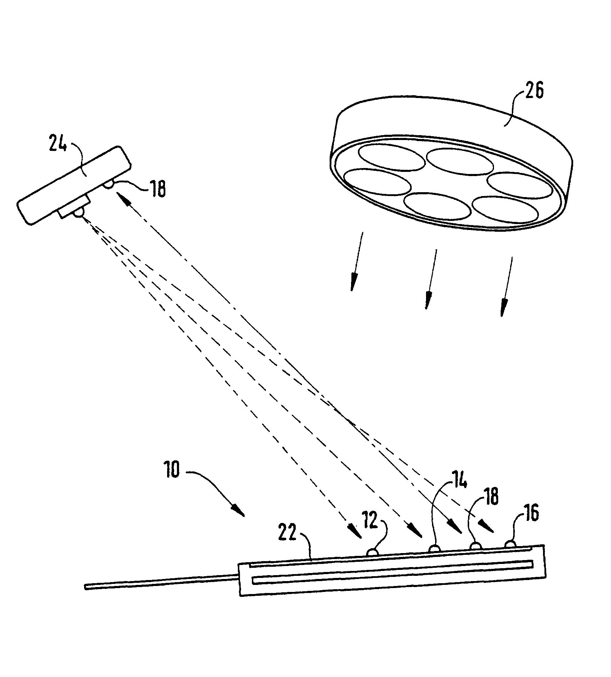

Ein Ausführungsbeispiel der Erfindung ist nachstehend unter Bezugnahme auf die zugehörige Zeichnung näher erläutert, die in der Figur schematisch ein aktives Instrument 10 darstellt. Das aktive Instrument 10 enthält Lichtquellen in Form von Leuchtdioden 12, 14, 16, die im Infrarotbereich strahlen und von zwei auf Infrarotstrahlung ansprechenden Kameras 24 beobachtbar sind. Aus von den Kameras 24 erfaßten Bildern kann durch Bildverarbeitung die Lage des Instrumentes 10 im Raum bestimmt werden.

Das Instrument 10, beispielsweise ein chirurgisches Instrument oder ein "Pointer", enthält weiterhin eine Einrichtung 18 zur drahtlosen Kommunikation, z. B. eine IR-Schnittstelle zur Datenübertragung. Damit kann das Instrument 10 identifiziert werden. Es ist auch möglich, das Instrument 10 drahtlos zu steuern, z.B. die Lichtquellen 12, 14 und 16 aus- und einzuschalten oder ihre Helligkeit zu verändern.

Die Stromversorgung für die Lichtquellen 12, 14 und 16 und für die Einrichtung 18 enthält einen Puffer für die Stromversorgung z. B. einen Kondensator 20. Der Puffer 20 wird von Solarzellen 22 aufgeladen, die ihrerseits die notwendige Energie von einer künstlichen Lichtquelle 26 oder aus dem Tageslicht beziehen.

## Patentansprüche

1. Aktives Instrument zur Positionsbestimmung bei Navigationssystemen zur Unterstützung chirurgischer Eingriffe,
**dadurch gekennzeichnet,**
**dass** das Instrument (10) wenigstens eine Solarzelle (22) zu seiner Stromversorgung aufweist.

2. Aktives Instrument nach Anspruch 1,
**dadurch gekennzeichnet,**
**dass** das Instrument (10) einen von der Solarzelle (22) gespeisten Pufferspeicher (20) zur Pufferung der Stromversorgung aufweist.

3. Aktives Instrument nach Anspruch 1 oder 2,
**dadurch gekennzeichnet,**
**dass** das Instrument (10) Lichtquellen (12, 14, 16) zur Positionsbestimmung des Instruments (10) aufweist, die von der Stromversorgung gespeist werden.

4. Aktives Instrument nach einem der Ansprüche 1 bis 3,
**dadurch gekennzeichnet,**
**dass** das Instrument (10) drahtlose Kommunikationsmittel (18) aufweist, die von der Stromversorgung gespeist werden.

## Claims

1. Active instrument for determining a position in navigation systems for assisting surgical operations,
**characterized in that** said instrument (10) comprises at least one solar cell (22) for its power supply.

2. Active instrument according to claim 1,
**characterized in that** said instrument (10) comprises a buffer storage means (20) being charged from said solar cell (22) for buffering said power supply.

3. Active instrument according to claim 1 or 2,
**characterized in that** said instrument (10) comprises light sources (12, 14, 16) for determining the position of said instrument (10), said light sources being fed from said power supply.

4. Active instrument according to one of claims 1 to 3,
**characterized in that** said instrument (10) comprises wireless communication means (18) being fed from said power supply.

## Revendications

1. Instrument actif de localisation pour systèmes de navigation facilitant les interventions chirurgicales,
**caractérisé en ce que** l'instrument (10) présente au moins une cellule solaire (22) pour l'alimentation en courant électrique.

2. Instrument actif selon la revendication 1,
**caractérisé en ce que** l'instrument (10) présente un réservoir d'accumulation (20) alimenté par la cellule solaire (22) afin d'égaliser l'alimentation en courant électrique.

3. Instrument actif selon les revendications 1 ou 2,
**caractérisé en ce que** l'instrument (10) possède des sources de lumière (12, 14, 16) pour la localisation de l'instrument (10), alimentés par l'alimentation en courant électrique.

4. Instrument actif selon l'une des revendications 1 à 3,
**caractérisé en ce que** l'instrument (10) est équipé de moyens de communication (18) sans fil, alimentés par l'alimentation en courant électrique.
